Europäisches Patentamt

European Patent Office

Office européen des brevets

(19)

(11) **EP 1 477 116 A1**

(12) **EUROPEAN PATENT APPLICATION**

(43) Date of publication:
**17.11.2004 Bulletin 2004/47**

(51) Int Cl.⁷: **A61B 5/06**, A61B 5/107

(21) Application number: **04076159.5**

(22) Date of filing: **15.04.2004**

| | |
|---|---|
| (84) Designated Contracting States:<br>**AT BE BG CH CY CZ DE DK EE ES FI FR GB GR**<br>**HU IE IT LI LU MC NL PL PT RO SE SI SK TR**<br>Designated Extension States:<br>**AL HR LT LV MK** | • **Squilla, John R. c/o Eastman Kodak Company**<br>**Rochester New York 14650-2201 (US)**<br>• **Boland, John T. c/o Eastman Kodak Company**<br>**Rochester New York 14650-2201 (US)**<br>• **Stephany, Thomas M.**<br>**c/o Eastman Kodak Company**<br>**Rochester New York 14650-2201 (US)** |
| (30) Priority: **29.04.2003 US 425249** | |
| (71) Applicant: **EASTMAN KODAK COMPANY**<br>**Rochester, New York 14650 (US)** | (74) Representative: **Haile, Helen Cynthia et al**<br>**Kodak Limited,**<br>**Patents Department (W92-3A),**<br>**Headstone Drive**<br>**Harrow, Middlesex HA1 4TY (GB)** |
| (72) Inventors:<br>• **Spoonhower, John P.**<br>**c/o Eastman Kodak Company**<br>**Rochester New York 14650-2201 (US)** | |

(54) **Probe position measurement to facilitate image registration and image manipulation in a medical application**

(57)     A method utilizing an imaging probe for registering images associated with a medical image processing application comprises the steps of: (a) providing a local tracking system that generates a field in a local area within which the imaging probe is used; (b) capturing first and second images representing substantially the same object; (c) sensing field emissions from the local tracking system to establish positional coordinates of the imaging probe during image capture; (d) using the positional coordinates to register the first and second images; and (e) utilizing the registered images to determine a characteristic of the object.

FIG. 1

EP 1 477 116 A1

## Description

The invention relates generally to the field of medical imagery, and in particular to the field of image registration as related to dental imagery.

Image registration in general is concerned with determining a precise geometric match between two or more images of the same object or area which are from different times or taken from different positions relative to the image content. In the present invention, the primary emphasis is on dental images (e.g., obtained from intra-oral digital imagery or dental radiography) taken on different dates or times. Comparison of such imagery, after registration, allows detailed analysis of any changes that may have occurred due to, e.g., new or larger cavities, bone loss, loosened fillings, etc.

The registration process often relies on tie points, which are points (image positions) of the same object in different images. Tie points must be accurately placed, and must be unambiguously identified. The tie points are then used to generate a polynomial function that is used to warp one image to another. Tie point selection can be an arduous process, requiring users to repeatedly cycle between overviews of the imagery and close-up views as they attempt to identify and then precisely indicate the common locations. The process of "zooming-in" and "zooming-out" can be time consuming, as well as disconcerting, frequently resulting in the user losing context, i.e., not being sure of which part of the image is being viewed.

In the aforementioned commonly assigned copending application Serial No. 09/970,243, entitled "Method for Registering Images in a Radiography Application", an image registration method is described for x-ray imagery, in which specific views of the x-rays are provided to optimize tie point selection accuracy and efficiency. The first view maintains context during initial point selection. The second view provides a detailed view of each point pair, to allow for fine adjustment, while automatically presenting each point pair in sequence to the user. After the tie points are refined and the images are registered, a third view is provided which allows direct comparison of the registered images.

Image registration thus is an important element in isolating historical changes in film or digital imagery. Change detection, in this context, is an image based concept, and refers to the process of comparing imagery over an area of interest taken at two different times. Images are compared either manually or automatically to determine those places where some change in the scene content has occurred. Imagery-based change detection can be performed on a variety of image types, including panchromatic, color, IR and multi-spectral image types. In some applications, the size, location and type of change can be determined.

Image registration is also an important element in three-dimensional modeling of intra-oral objects, e.g., in effecting imagery of a prepared cavity in a tooth followed by automatic generation of a model to control automatic fabrication of a dental inlay for the cavity. For instance, U.S. Patent No. 4,837,732 (Brandestini et al) describes a method for a dentist to record the shape in situ of teeth prepared for repair. The method involves the acquisition of data defining the three-dimensional shape of prepared teeth and their immediate vicinity. First, a video display shows a live image from a scan head, and the scan head is manually oriented relative to the prepared teeth while observing the image of the teeth on the video display. Thereafter the data produced by the scan head in a selected orientation generates corresponding depth and contrast images, and a depth image is processed based on the contrast image. This method also includes the step of superimposing graphic markers on the image displayed on the video display to facilitate an on-line alignment of the teeth displayed in the live image with reference data from previous data acquisitions.

The drawback to this method from the prior art is that it incorporates a registration scheme that can later interfere with the quality of the results, and also requires that the dentist be able to hold the scan head almost perfectly still at a specific point in the procedure. More specifically, the artifacts typically due to the 3D registration scheme (such as fringe, speckle and/or venetian blind effect) are cited in the patent as "intolerable and must be eliminated" since phase angle differences are used for measurement of the depth. Furthermore, the patent cites a need for a "quasi-instantaneous 3D acquisition following a trigger release", the essential condition being that the orientation of the scan head must not change between the search and acquisition modes.

In the aforementioned commonly assigned copending application Serial No. 09/894,627, entitled "Method and System for Creating Models from Imagery", a method is described for creating dental models from imagery in which errors due to the lack of certainty of knowledge about the image positions is addressed by using a method of analytical adjustment to control points. Note that image position is used here to denote both the position and the orientation of the sensor during image formation. According to this method, creating a dental model from a series of images of an intra-oral object includes the steps of (a) capturing a series of images of an intra-oral object from a plurality of capture positions, where the object includes common surface features and a control target arranged with respect to the object to provide control features; (b) measuring the common features from the series of images of the object and the control features from the control target imaged with the images of the object; (c) analytically generating a 3-dimensional model of the object by photogrammetrically aligning the measurements of the control features, thereby reducing image errors due to the variability of the capture positions; and (d) adjusting the photogrammetrically aligned 3-dimensional model of the object by aligning the common features of the model to like features on the image of the object, thereby producing an aligned dental model from the series of images.

Employing a mensuration method that utilizes photogrammetric projection, the principal advantage of the method described in application Serial No. 09/894,627 is that the use of photogrammetric projection methods and adjustment to control eliminates the need for a conventional registration scheme, such as that used in Brandestini et al, which projects stripes of light onto the target and can result in unacceptable artifacts.

Notwithstanding these advantages, a robust registration procedure would remain a useful alternative. What is needed is a system that provides accurate knowledge of the sensor position and orientation during image formation. This knowledge can be used to either eliminate the need for manual provision of tie points, or for the adjustment to control described above, to provide more reliable initial position estimates to that process.

The present invention is directed to overcoming one or more of the problems set forth above. Briefly summarized, according to one aspect of the present invention, a method utilizing an imaging probe for registering images associated with a medical image processing application comprises the steps of: (a) providing a local tracking system that generates a field in a local area within which the imaging probe is used; (b) capturing first and second images representing substantially the same object; (c) sensing field emissions from the local tracking system to establish positional coordinates of the imaging probe during image capture; (d) using the positional coordinates to register the first and second images; and (e) utilizing the registered images to determine a characteristic of the object.

In yet another aspect of the invention, an image processing system utilizing an imaging probe for registering images associated with a medical image processing application comprises: a local tracking system that generates a field in a local area; an imaging probe utilized within the local area for capturing first and second images representing substantially the same object; one or more sensors associated with the imaging probe for sensing field emissions from the local tracking system to establish positional coordinates of the imaging probe during image capture; and one or more processing stages using the positional coordinates to register the first and second images, said one or more processing stages utilizing the registered images to determine a characteristic of the object.

With accurate knowledge of the sensor position and orientation during image formation, the advantage of the invention is that manual provision of tie points is eliminated. In addition the adjustment to control described in connection with the prior art can be used to refine the position estimates, thereby providing more reliable initial position estimates for the subtractive process.

These and other aspects, objects, features and advantages of the present invention will be more clearly understood and appreciated from a review of the following detailed description of the preferred embodiments and appended claims, and by reference to the accompanying drawings.

FIG. 1 is an illustration of a dental office outfitted according to the invention to capture and record local positioning information along with imagery of an intra-oral object.

FIG. 2 is a perspective diagram of a computer system that is useful in practicing the present invention.

FIG. 3 shows an intra-oral imaging probe and display system that is useful in practicing the present invention.

FIG. 4 shows a block diagram of the electronics in the integral base associated with the imaging probe shown in Figure 3.

FIG. 5 is an illustration of imagery performed according to the invention on the lower jaw and teeth of a typical patient by use of a digital intra-oral imaging probe.

FIG. 6 is a an illustration of imagery performed according to the invention on the lower jaw and teeth of a typical patient by use of an x-ray source.

FIG. 7 shows a block diagram of the various stages of a registration method according to the invention.

FIG. 8 is an illustration of a standard analytical photogrammetric technique which uses a sensor geometry model to project from an image space to an object space for one image, and then to project from an object space to an image space for another image.

FIG. 9 is a block diagram of a subtractive process that is performed on the images, once the projective process illustrated in Figure 8 is completed.

Because image registration systems employing tie points are well known, the present description will be directed in particular to attributes forming part of, or cooperating more directly with, a method and system in accordance with the present invention. Method and system attributes not specifically shown or described herein may be selected from those known in the art. In the following description, a preferred embodiment of the present invention would ordinarily be implemented as a software program, although those skilled in the art will readily recognize that the equivalent of such software may also be constructed in hardware. Given the system as described according to the invention in the following materials, software not specifically shown, suggested or described herein that is useful for implementation of the invention is conventional and within the ordinary skill in such arts. If the invention is implemented as a computer program, the program may be stored in conventional computer readable storage medium, which may comprise, for example; magnetic storage media such as a magnetic disk (such as a floppy disk or a hard drive) or magnetic tape; optical storage media such as an optical disc, optical tape, or machine readable bar code; solid state electronic storage devices such as random access memory (RAM), or read only memory (ROM); or any other physical device or medium employed to store a computer program. The computer program could also be made available to the operator's computer via a network; the use of the program could be provided as a service for which the operator pays a fee.

Before describing the present invention in detail, it is helpful to understand that the present invention is preferably utilized on any well-known computer system, such a personal computer. Consequently, the computer system will not be discussed in detail herein. It is also instructive to note that the images are either directly input into the computer system (for example, from a digital intra-oral imaging probe or a digital radiographic source) or digitized before input into the computer system (for example, by scanning an original, such as a silver halide x-ray film or other form of radiographic image).

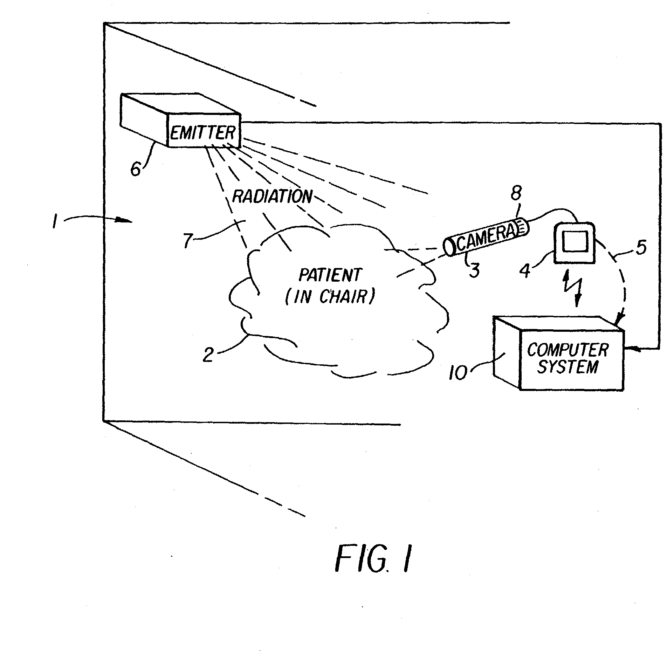

Referring first to Figure 1, the basic concept of the invention is illustrated in relation to a dental office 1 incorporating a predetermined, constrained patient location 2, e.g., a conventional dental chair with head restraints, where a patient is positioned for a dental procedure. In connection with such a procedure, and according to one aspect of the invention, dental imagery is captured by an intra-oral imaging probe 3 connected to a hand held display unit 4, as is disclosed in co-pending, commonly assigned U.S. Patent Application Serial No. 09/796,239, entitled "Intra-Oral Camera with Integral Display" and filed 28 February 2001 in the names of J. P. Spoonhower, J. R. Squilla and J. T. Boland. The display unit 4 includes a transceiver for communicating image data to a computer system 10. Alternatively, the display 4 (or the imaging probe 3) could be physically tethered to the computer 10, as shown by the dotted connection 5, in order to transfer the captured and/or processed image data from the display 4 (or imaging probe 3) to the computer system 10.

In accordance with the invention, a local real time tracking system 6 is fixedly located as a base unit in the room for emitting a field 7 generally in the direction of the imaging probe 3 and the patient location 2. One or more miniature field sensors 8 are incorporated into the handheld imaging probe 3 in order to sense the field emitted by the base unit of the local tracking system 6. This will give the location of the probe 3 for each image relative to the base unit. In addition, an additional sensor 8 may be placed in the mouth of the patient to give the position of the probe 3 relative to the mouth and the base unit of the local tracking system 6. From this sensed information, which is transferred to the computer 10 for processing, it is possible to record the location and orientation of the imaging probe while images of the oral cavity are captured by the imaging probe 3 and recorded. Thus, the system provides a means for recording evidence of imaging probe position with a high degree of accuracy and precision. Imaging probe position can be recorded with the captured images as location and orientation metadata, and thereby used to implement an image registration process to facilitate subtraction and other processing of the captured images. For example, images of the same portion of the mouth taken at different times (perhaps in sequential visits to a dentist) could be more easily registered and subtracted to improve the process of rendering difference images critical to identifying changes in the images, such as bone loss.

The local tracking system 6 may be a receiver for a conventional global positioning system (GPS) that is commercially available for local applications. Such systems are well-known, and may be incorporated in a hybrid installation with pseudolites to improve reception within a building (see, for example, U.S. Patent No. 5,686,924, entitled "Local-Area Position Navigation System with Fixed Pseudolite Reference Transmitters" and which issued 11 November 1997). Pseudolites can be the basis for an entirely independent navigation system, see for example the article by E. A. LeMaster and S. M. Rock entitled "A Local-Area GPS Pseudolite-Based Mars Navigation System", *IEEE 10th International Conference on Advanced Robotics,* Budapest, Hungary, August 2001, pp. 1- 6. It is also known to use localized real time tracking systems, such as described in International Patent Application WO 01/89405 A1, entitled "Fully-Automatic, Robot-Assisted Camera Guidance Using Position Sensors For Laparoscopic Interventions", published 29 November 2001. One commercially available real-time tracking system is the miniBIRD™ tracking system offered by Ascension Technology Corporation, Burlington, Vermont. The field generated by the local tracking system 6 may be field radiation of whatever form is suitable under the circumstances; for example, because of the enclosed space of the dental office 1, the emitted radiation may be a magnetic field emission, such as provided by the miniBIRD™ tracking system, and the field sensor would therefore be a magnetic field sensor. Alternatively, the emitted field may be a radio-frequency electromagnetic emission, such as provided by a GPS or a pseudolite transmitter, and the field sensor would therefore be an RF field sensor.

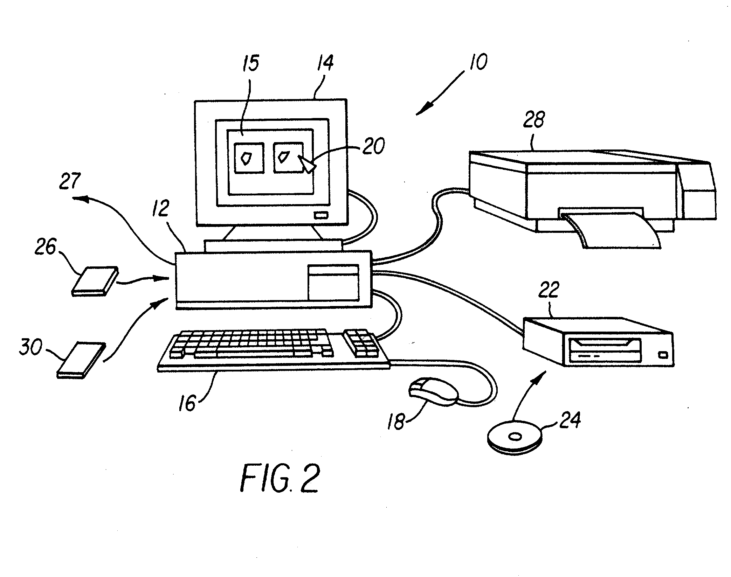

Referring to Fig. 2, there is illustrated a typical configuration of the computer system 10 for implementing aspects of the present invention. Although the computer system 10 is shown for the purpose of illustrating a preferred embodiment, the present invention is not limited to the computer system 10 shown, but may be used on any electronic processing system. The computer system 10 includes a microprocessor-based unit 12 for receiving and processing software programs and for performing other processing functions. A display 14 is electrically connected to the microprocessor-based unit 12 for displaying user-related information associated with the software, e.g., by means of a graphical user interface (GUI) 15. A keyboard 16 is also connected to the microprocessor based unit 12 for permitting a user to input information to the software. As an alternative to using the keyboard 16 for input, a mouse 18 may be used for moving a selector (cursor) 20 on the display 14 and for selecting an item on which the selector 20 overlays, as is well known in the art.

A compact disk-read only memory (CD-ROM) 22 is connected to the microprocessor based unit 12 for receiving software programs and for providing a means of inputting the software programs and other information to the microprocessor based unit 12 via a compact disk 24, which typically includes a software program. In addition, a floppy disk 26 may also include a software program, and is inserted into the microprocessor-based unit 12 for inputting the software program. Still further, the microprocessor-based unit 12 may be programmed, as is well known in the art, for storing the software program internally. The microprocessor-based unit 12 may also have a network connection 27, such as a telephone line, to an external network such as a local area network or the Internet. Accordingly, the software program may be received over the network, perhaps after authorizing a payment to a network site. A printer 28 is connected to the microprocessor-based unit 12 for printing a hardcopy of the output of the computer system 10.

Images may also be displayed as part of the graphical user interface 15 on the display 14 via a personal computer card (PC card) 30, such as, as it was formerly known, a PCMCIA card (based on the specifications of the Personal Computer Memory Card International Association) which contains digitized images electronically embodied in the card 30. The PC card 30 is ultimately inserted into the microprocessor based unit 12 for permitting visual display of the image on the display 14. Images may also be input via the compact disk 24, the floppy disk 26, or the network connection 27. Any images stored in the PC card 30, the floppy disk 26 or the compact disk 24, or input through the network connection 27, may have been obtained from a variety of sources, such as a digital intra-oral imaging probe 3 or an x-ray image scanner (not shown).

The invention is useful in a subtractive radiography process where change detection is used to identify areas of differences among images of the same region that were collected at different times. Registration of the images is a prerequisite for the change detection process. In accordance with the invention, an image capture position and orientation system for the purpose of facilitating both image registration and extraction of 3D data is described. When used in visible medical imaging (recording and analyzing photographic images), this system facilitates both the imaging and the re-construction of the 3-D topology of an object, such as a tooth, when visible light is sensed in conjunction with the capture position data. Additionally, by using the invention described herein, a capture position and orientation system, in conjunction with the invention described in the aforementioned commonly assigned copending application Serial No. 09/970,243, would allow recording temporal differences in both optical and x-ray images.

The image capture position and orientation measurement system would facilitate the registration of images and enable a more automatic version of the software assisted process described in the aforementioned commonly assigned copending application Serial No. 09/970,243 to be applied. For example, this capability could be used to map surface wear in a tooth if a patient had a grinding bite, or to monitor bone loss through periodontal disease in a patient.

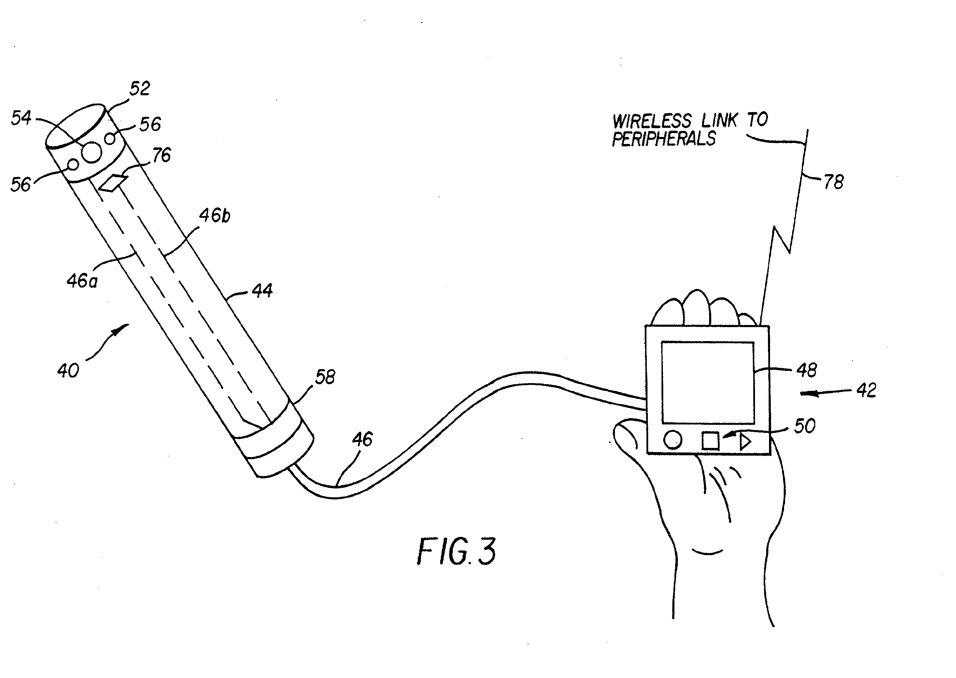

The invention incorporates and modifies any conventional image capture system, such as a conventional intra-oral imaging probe or an x-ray or ultrasound source. A preferred implementation is a imaging probe of the type disclosed in the aforementioned U.S. Patent Application Serial No. 09/796,239, entitled "Intra-Oral Camera with Integral Display". Referring to Figure 3, an intra-oral dental imaging probe system of the type disclosed in the above application includes a portable dental imaging probe 40 and a power source, illumination source and a display unit integrally located in a portable enclosure (hereinafter referred to as the integral base 42) tethered to the imaging probe 40. The imaging probe 40 and the integral base 42 thus constitute an intra-oral imaging probe with integral display. The dental imaging probe 42 includes a handpiece 44 and a cable 46 connecting the dental imaging probe 40 to the integral base 42. As shown for illustrative purposes in Figure 3, the integral base 42 can be easily cradled in a hand, and includes a display monitor 48 that can be easily hand positioned relative to the dentist's and/or patient's line of sight. A set of user controls 50 are provided on the integral base 42 that can be easily hand-navigated for controlling the illumination and the images displayed on the monitor, as well as communicating with peripheral devices. The handpiece 44 supports a removable lens unit 52 that includes a lens 54 and light emitting apertures 56. The handpiece 44 is generally elongated and cylindrical with a central axis. The lens 54 is positioned to receive light impinging on the handpiece in a direction substantially perpendicular and normal to the central axis of the handpiece. In accordance with the invention, one or more field sensors 58 are located on the handpiece 44 to sense the field emissions from the local tracking system 6. Despite this preferred implementation, it should be clear that the invention can be implemented on many other types of imaging probes, including imaging probes of various shapes and capabilities, and without any portable or attached display capability.

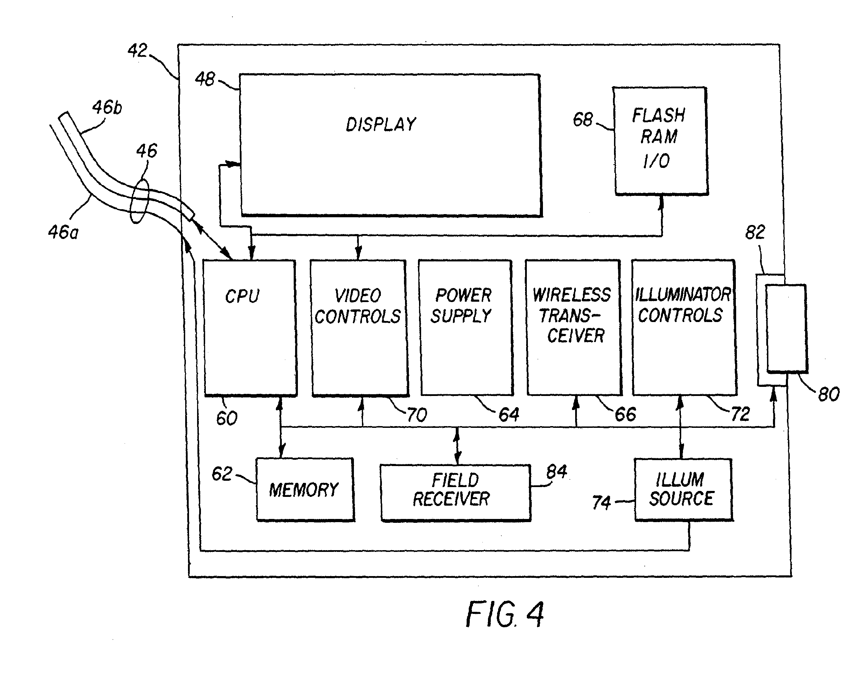

Referring to Figure 4, the integral base 42 in the preferred implementation includes a central processing unit (CPU) 60, a CPU memory 62, a power supply 64, a wireless transceiver 66, and flash memory (RAM) 68. The user controls 50 interface with a video control unit 70 and an illuminator control unit 72. The illuminator control unit 72 connects with an illumination source 74, which provides illumination to the handpiece 44 through a fiber optic 46a that is part of the cable 46. The illumination source may take a variety of forms known to those of skill in this art, such as a halogen arc lamp lighting system or a tungsten/halogen lamp. The power supply 64 is connected by a power cable (not shown) to a power source, such as a wall socket. The image signal communication between the handpiece 44 and the CPU 60 is maintained through an electrical connection 46b, which is also in the cable 46. While not shown in detail, the handpiece 44 also supports a connection of the fiber optic 46a with the light emitting apertures 56 and a connection of the electrical conductor 46b to an image sensor 76, such as a conventional charge coupled device (CCD), and the field sensor(s) 58. The image sensor 76 is arranged in a conventional optical path, with mirrors and other optical components as might be necessary, such that the lens 54 can form an image of an intra-oral object on the image sensor 76. The field signals from the field sensor(s) 58 are transferred via the electrical connection 46b to a field receiver 84 in the integral base 42.

It should be noted that portability is facilitated by incorporating into the dental imaging probe system both a high quality image display 48 along with means to transfer image data to a physically separate and distinct data storage associated with an image printing capability. The means to accommodate a transfer of image data may include (a) wireless RF or microwave transceiver technology, (b) wireless infra-red transmission technology, and/or (c) removable memory technology embodied in physically small elements 80, such as flash RAM cards or small hard drives, that are easily removed from an interface 82 in the imaging probe part of the system and subsequently plugged into either the image data storage or printer parts of the system.

Accordingly, the dental imaging probe system can, through the transceiver 66 in its integral base 42, initiate communication via wireless links 78 with a variety of peripheral units. Each of these units would have its own data storage for receiving the transmitted images. Without intending to be exhaustive as to type of peripheral unit that may be accessed, such peripheral units include a larger monitor or television receiver, a printer, and a computer system, such as any conventional desktop PC, where the images may be processed and stored. With this arrangement, a dental practitioner may view an image on the integral base 42 and immediately initiate its transfer to any one of the peripheral units by means of the user controls 50. The incorporation of the transceiver 66 and the display monitor 48 into the dental imaging probe system further enables the practitioner to view the results of an image recording, and conveniently display the captured image(s) either for the practitioner's or patient's benefit. For this purpose, the transceiver 66 would receive images from a storage peripheral, such as a computer system, and display the stored images on a display monitor. Importantly, such viewing occurs without the requirement of producing a physical print of the image.

In operation, the handpiece 44 is maneuvered into the region of the patient location 2, where it is exposed to the field emissions 7 from the local tracking system 6. The field sensor(s) 58 on the handpiece 44 senses the presence of the field 7 and registers a signal that is transmitted over the electrical conductor 46b to the field receiver 84 in the integral base 42. The field receiver 84 detects and converts the field emissions received by the field sensor into field measurements that are sent to a suitable peripheral unit, such as a computer, for processing (alternatively, the processing may occur within the integral base 42 or within the tracking system 6). Basically, the local tracking system 6 tracks the location of the one or more field sensors 58 in the designated field 7. The tracking occurs in real time, with six degrees-of-freedom - three position coordinates and three orientation angles. (This tracking technique is based on well known technology exemplified by systems such as the aforementioned commercially available miniBIRD™ tracking system offered by Ascension Technology Corporation). Using the known position of the sensor(s) 58, the coordinates of the imaging probe 3 within the designated field space can be determined, and this coordinate data is included and stored with the captured images as metadata. Using the metadata, the position of the images captured by the imaging probe 3 can be inferred from these coordinates.

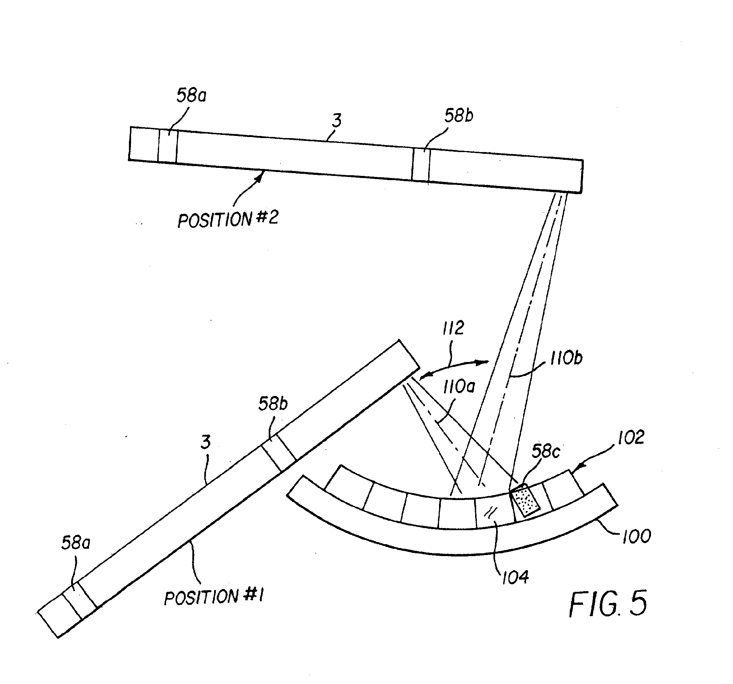

Although there are applications of the present invention throughout medical imaging, consider for example, an application in dentistry. Fig. 5 shows the lower jaw 100 and teeth 102 of a typical patient, and an imaging probe 3 having means for both image recording and position/orientation detection. The imaging probe 3 is shown taking an image of tooth 104 from a first position #1. That image is saved and then a second image is taken by moving the imaging probe 3 to a second position #2. Then, conventional software may be used to register the two (or more) images using the position data captured at the time of exposure (and stored as metadata with the images) so that a proper overlap of the two images is obtained. Given the proper registration of the two images, a calibration process enables accurate derivation of the distances involved in measuring the intra-oral object (e.g., the tooth 104). Figure 5 also illustrates the use of two field sensors, a first field sensor 58a and a second field sensor 58b. The position information obtained by the tracking system 6 for the two sensors enables the two different aspects of the projected images to be obtained. That is, the image at the first position #1 has a projection aspect determined by its axis of view 110a and its angular relationship to the object and the second position #2 has a projection aspect determined by its axis of view 110b and its angular relationship to the object. Knowing the position coordinates of the two sensors 58a and 58b enables the angular relationship 112 to be determined between the two images and from that aspect information the two pictures can be adjusted, e.g., by conventional morphing techniques, such that they precisely overlap.

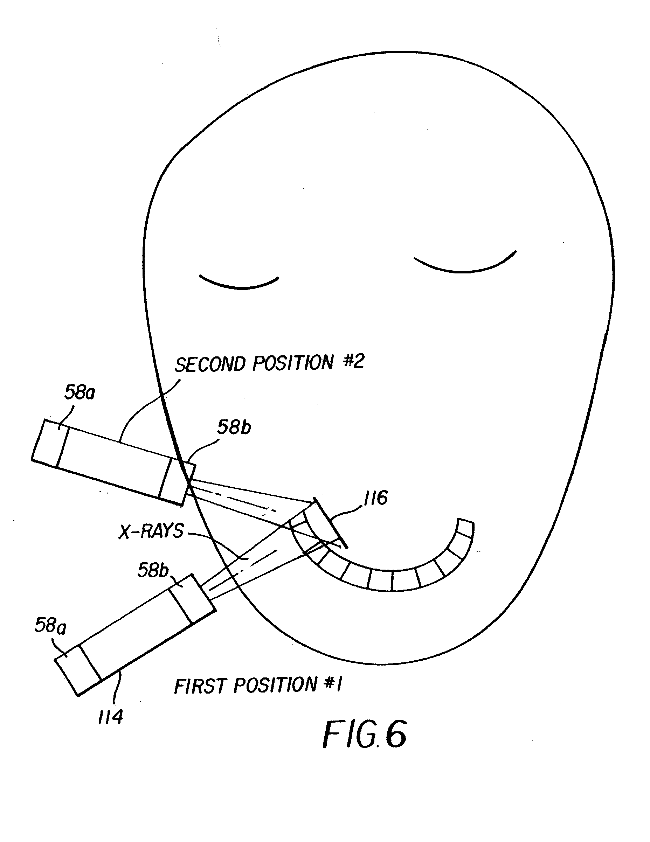

Referring to Figure 6, in the case of an X-ray investigation, an x-ray source 114 for recording x-ray images is shown in two different positions #1 and #2. The x-ray source 114 has attached field sensors 58a and 58b to record the field information, from which the position/orientation of the source 114 is determined. This system can be used with a photosensitive receiver 116, in this case either a frame of x-ray film or a digital radiographic sensor, to capture the image information. As was described in connection with Figure 5, software would again normalize the differences in position of the x-ray emissions from the two positions of the x-ray source 114, thus enabling accurate registration of the images. Since the film or the sensor would be held in fixed orientation to the tooth, only position information of the source relative to the tooth would be required to calibrate the system.

In another embodiment of the invention, another field sensor 58c (see Figure 5) may be placed on a patient's tooth (or jaw) for instantaneous monitoring of the actual position coordinates of the tooth (or jaw) position. This would further enable the calibration process by allowing an accurate derivation of the distance between the probe and the tooth (or jaw), through calculation of the difference in x,y,z coordinates in reference to the magnetic field. This approach has the further advantage of simplifying the image registration process by tracking, and thereby allowing the elimination of, any movement between the images of the tooth relative to the probe. The registration process is simplified due to identification and removal of rotation/translation of the tooth relative to the probe. In yet another extension of the concepts embodied in the present invention, a fine mechanical tip may be formed on the imaging probe 3 or x-ray source 114, which contacts the object to be recorded at a specific point. Then, by using the aforementioned methodology of the invention, the x,y,z coordinates of that specific point may be determined and recorded in reference to the magnetic field.

An advantageous use of the invention is in conjunction with the methodology described in the aforementioned commonly assigned copending application Serial No. 09/970,243, where custom software described in that application would allow recording temporal differences in both optical and x-ray images. The process described in Serial No. 09/970,243 includes the use of manually selected tie points (seed points) followed by autocorrelation to find additional tie points, leading to the warping of one image to another which necessarily includes an image resampling step. The image capture position and orientation measurement system described according to the present invention would simplify and improve the subtractive radiography process described in Serial No. 09/970,243, thus facilitating the registration of images and applying a more automatic version of the software assisted process described in that application.

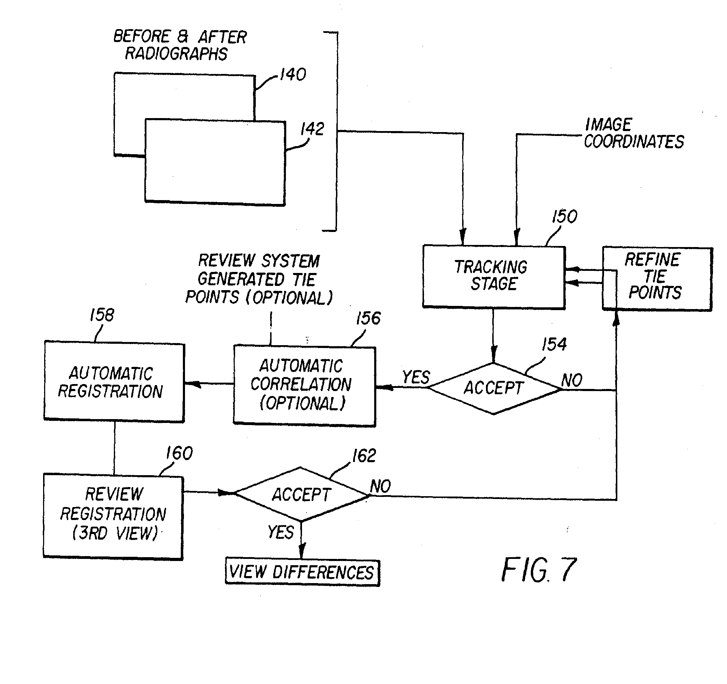

Figure 7 shows an automated method for placing reference points in a radiography application, which is based on a version of the method shown in Serial No. 09/970,243 that has been modified according to the current invention. The method is shown in its several stages in Figure 7 for the two images 140 and 142, which represent before and after dental images or radiographs of an oral object, such as a tooth ("before" and "after" is meant to represent a time sequence that would reveal, e.g., changes in the tooth or bone structure caused by a cavity or disease). Using the coordinates developed by the tracking system 6, the images are processed in a tracking stage 150 to locate potential tie points. The images may be presented to the user via the graphical user interface 15, whereupon the user may signal an acceptance decision 154 through manipulation of the mouse 18 or the keyboard 16 (if, for any reason, the results are unacceptable, the process is returned to a manual refinement stage, not shown, until the result is acceptable). The result is a set of refined, automatically-determined tie points that are suitable for the registration process.

Once accepted, the refined tie points can be used in conjunction with optional automatically correlated points in the correlation stage 156. These optional points may then be reviewed by the user. In the auto registration stage 158, a polynomial function is generated to relate the tie points. In its simplest form, the polynomial (alignment equation) is of the form

$$X = a_1 + a_2 X' + a_3 Y'$$

with only three constants (and a similar equation for Y). Hence, locating three reference (tie) points that are common to two sequential images allows one to be rotated and stretched (warped) to align with the other. (See pages 201 - 208 on Alignment in The Image Processing Handbook, Second Edition, by John C. Russ, CRC Press, 1995). Typically, more tie points are involved in the registration process. For instance, in commonly-assigned U.S. Patent No. 6,163,620 (entitled "Automatic Process for Detecting Changes Between Two Images"), which is incorporated herein by reference, between five and one hundred tie points are used. The polynomial function is then used in the auto registration stage 158 to warp the right image 142 to the left image 140 (or vice versa). Once registration is completed, the results are aligned side by side for review in the registration review stage 160. Known alignment techniques may be employed to render the left and right images for this view with the same zoom level and image centering. (cf., The Image Processing Handbook). If the user deems the registration adequate, acceptance is signaled by the acceptance decision 162 through manipulation of the mouse 18 or the keyboard 16.

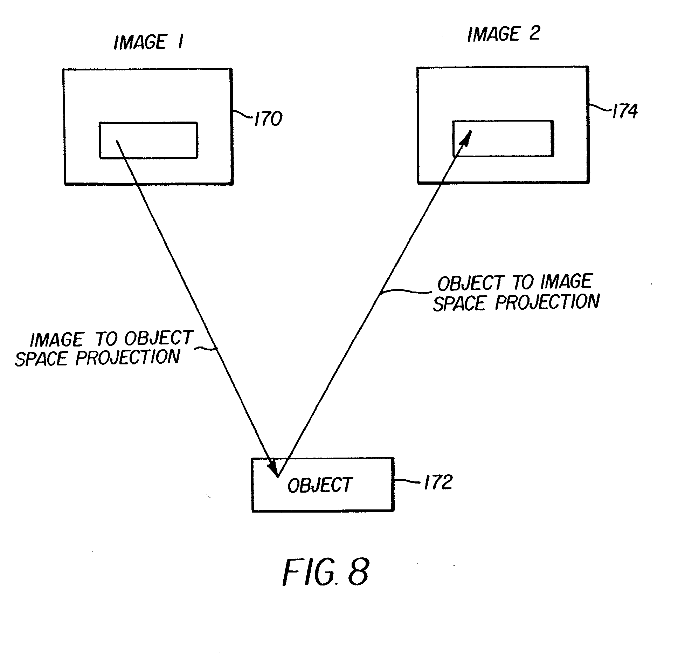

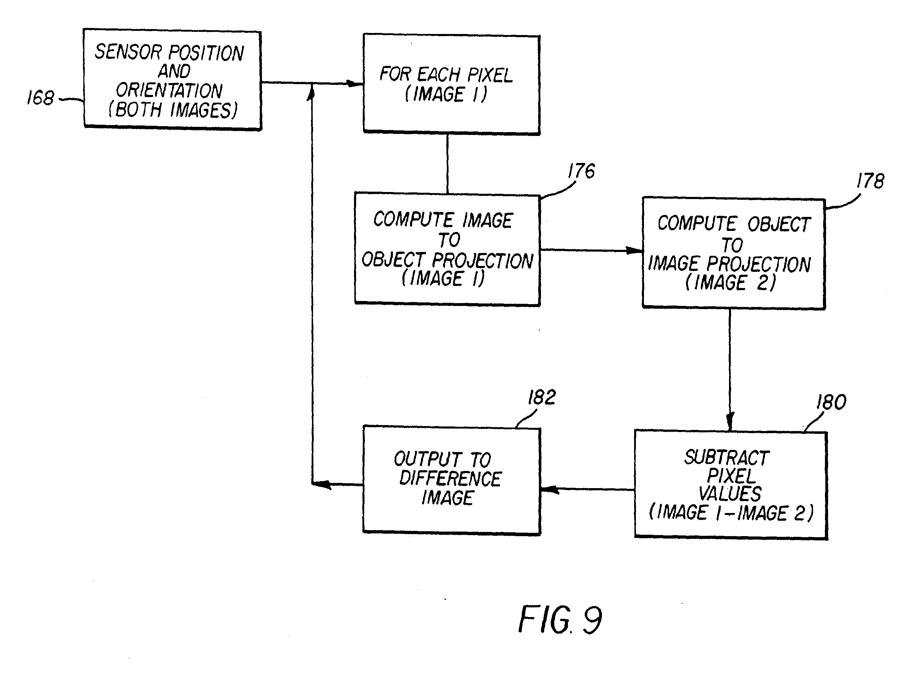

The use of the field sensor(s) 58 provides the precise position and attitude of the sensor for each image. This knowledge allows a suitable analytical geometry model for the specific sensor(s) to be used to predict the corresponding position of each pixel from one image to another. This is a standard analytical photogrammetric technique (see Figure 8 and 9) which uses the sensor geometry model (providing data 168 of the sensor position and orientation during image formation) to project (stage 176) from the image space 170 (for image 1) to object space 172, and then to project (stage 178) from object space 172 to image space 174 (for image 2). This projective process precludes the need for manually selected tie points (seed points), minimizes, and could in some instances eliminate, the need for autocorrelation, and eliminates the need for an image warping step; along with the associated resampling. Once the projective process determines the corresponding pixel in the second image, the subtractive process (stage 180) is performed, directly yielding a difference image 182 (see Figure 9).

Another application of the positioning system of the invention is found in the aforementioned commonly assigned copending application Serial No. 09/894,627, entitled "Method and System for Creating Models from Imagery", in which a method was described for creating dental models from imagery in which errors due to the lack of certainty of knowledge about the image positions were addressed using a method of analytical adjustment to control points. Note that image position is used here to denote both the position and the orientation of the sensor during image formation. That method corrects the errors by analytically projecting a known 3-D model into an existing image or multiple images, assuming initial estimates for the image positions, then determining the misalignment of the control points between the model and the image, and refining the estimates of image position through photogrammetric adjustment. The projection is an analytical process, meaning that it is accomplished mathematically, and the determination of misalignment may be accomplished interactively or automatically with appropriate image processing algorithms.

The current invention employs a position determination system, which provides knowledge of the sensor position and orientation during image formation. This knowledge can be used to either eliminate the need for the adjustment to control described above, or to provide more reliable initial estimates to that process. In the former case, the entire process is made more efficient by eliminating the need for interactive or automatic control point measurement followed by photogrammetric adjustment. In the latter case, the adjustment process is enhanced through the use of improved initial estimates, which allows the process to be initialized in a more accurate state, i.e., closer to the true condition.

Adjustment should be understood as a process that includes adjustment to control—as well as adjustment to conjugate measurements—which will work very well when there are excellent starting estimates (as would be obtained from the position sensors). In such a case, the positional estimates can be used to automatically look for tie points via software by (a) using the software to locate a definable point in a first image (b) projecting the position of that point into a second image (c) searching for the exact conjugate point in the second image, thereby producing a set of tie points, and (d) repeating these steps until the required number of tie points are obtained. Then, adjustment can be made to the tie points.

**Claims**

1. A method utilizing an imaging probe for registering images associated with a medical image processing application, said method comprising the steps of:

   (a) providing a local tracking system that generates a field in a local area within which the imaging probe is used;
   (b) capturing first and second images representing substantially the same object;
   (c) sensing field emissions from the local tracking system to establish positional coordinates of the imaging probe during image capture;
   (d) using the positional coordinates to register the first and second images; and
   (e) utilizing the registered images to determine a characteristic of the object.

2. The method as claimed in claim 1 wherein the step (d) of using the positional coordinates comprises the steps of:

   using the positional coordinates of the imaging probe to establish reference points for the first and second images; and
   registering the first and second images by utilizing the reference points of the images.

3. The method as claimed in claim 1 wherein the step (d) of using the positional coordinates comprises the steps of:

   using the positional coordinates to establish an analytical geometry model for the imaging probe; and
   using the analytical model to predict a corresponding position of each pixel in the second image from its location in the first image;
   given the location of the corresponding pixels in the second image, utilizing a subtractive process to yield a difference image that is used in step (e) to determine a characteristic of the object.

4. The method as claimed in claim 1 wherein the positional coordinates denote both the position and the orientation of the imaging probe.

5. The method as claimed in claim 1 wherein the positional coordinates are included with the images as metadata.

6. The method as claimed in claim 1 wherein the determined characteristic of the object is a dimensional characteristic that is used in a subtractive process.

7. The method as claimed in claim 1 wherein one or more sensors are affixed to a device for capturing the images in step (b), and wherein the field emissions sensed in step (c) determine for each image the position of the device relative to the tracking system.

8. The method as claimed in claim 7 wherein one or more sensors are also affixed to a patient undergoing a medical process, and wherein the field emissions sensed in step (c) further determine for each image the position of the device relative to the patient and the tracking system.

FIG. 1

FIG.2

FIG.3

WIRELESS LINK TO PERIPHERALS

FIG. 4

FIG. 5

FIG.6

*FIG. 7*

BEFORE & AFTER RADIOGRAPHS

140

142

IMAGE COORDINATES

150 TRACKING STAGE

REFINE TIE POINTS

154 ACCEPT — NO / YES

REVIEW SYSTEM GENERATED TIE POINTS (OPTIONAL)

156 AUTOMATIC CORRELATION (OPTIONAL)

158 AUTOMATIC REGISTRATION

160 REVIEW REGISTRATION (3RD VIEW)

162 ACCEPT — NO / YES

VIEW DIFFERENCES

IMAGE I

IMAGE 2

170

174

OBJECT TO IMAGE
SPACE PROJECTION

IMAGE TO OBJECT
SPACE PROJECTION

OBJECT

172

FIG. 8

FIG. 9

**European Patent Office**

## EUROPEAN SEARCH REPORT

Application Number

EP 04 07 6159

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 02/00093 A (YAGEL RONI ; INSIGHTEC IMAGE GUIDED TREAT L (IL)) 3 January 2002 (2002-01-03) * paragraph [0008] - paragraph [0034]; figure 1 * | 1,2,4-8 | A61B5/06 A61B5/107 |
| X | US 6 402 707 B1 (ERNST MAURICE M) 11 June 2002 (2002-06-11) * the whole document * | 1,2,4-8 | |
| X | US 2002/176541 A1 (ZEISS MARIO ET AL) 28 November 2002 (2002-11-28) * paragraph [0015] - paragraph [0028]; claim 8; figure 1 * | 1,2,7,8 | |
| A | US 5 113 424 A (DESJARDINS PAUL J ET AL) 12 May 1992 (1992-05-12) * abstract * * column 9, line 3 - column 10, line 54; figure 1 * | 1-4,6-8 | |
| A | US 2002/077542 A1 (VILSMEIER STEFAN ET AL) 20 June 2002 (2002-06-20) * paragraph [0036] - paragraph [0046]; figure 1 * | 1-4,7,8 | TECHNICAL FIELDS SEARCHED (Int.Cl.7) A61B |
| A | WO 02/096261 A (PERSKY NATHAN ; DENX AMERICA INC (US)) 5 December 2002 (2002-12-05) * abstract * * page 10, line 22 - page 17, line 2 * | 1,2,4,7,8 | |

The present search report has been drawn up for all claims

| Place of search | Date of completion of the search | Examiner |
|---|---|---|
| Munich | 20 September 2004 | Artikis, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 1 477 116 A1

## ANNEX TO THE EUROPEAN SEARCH REPORT
## ON EUROPEAN PATENT APPLICATION NO.

EP 04 07 6159

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2004

| Patent document cited in search report | | Publication date | Patent family member(s) | | Publication date |
|---|---|---|---|---|---|
| WO 0200093 | A | 03-01-2002 | AU | 6779901 A | 08-01-2002 |
| | | | WO | 0200093 A2 | 03-01-2002 |
| US 6402707 | B1 | 11-06-2002 | AU | 6684301 A | 08-01-2002 |
| | | | CA | 2413142 A1 | 03-01-2002 |
| | | | EP | 1296592 A1 | 02-04-2003 |
| | | | JP | 2004502137 T | 22-01-2004 |
| | | | NZ | 523827 A | 25-06-2004 |
| | | | WO | 0200115 A1 | 03-01-2002 |
| | | | US | 2002143276 A1 | 03-10-2002 |
| US 2002176541 | A1 | 28-11-2002 | EP | 1260179 A1 | 27-11-2002 |
| | | | AT | 235187 T | 15-04-2003 |
| | | | DE | 50100132 D1 | 30-04-2003 |
| | | | ES | 2194809 T3 | 01-12-2003 |
| US 5113424 | A | 12-05-1992 | NONE | | |
| US 2002077542 | A1 | 20-06-2002 | EP | 1219260 A1 | 03-07-2002 |
| | | | AT | 243473 T | 15-07-2003 |
| | | | DE | 50002672 D1 | 31-07-2003 |
| | | | ES | 2199737 T3 | 01-03-2004 |
| WO 02096261 | A | 05-12-2002 | EP | 1401323 A2 | 31-03-2004 |
| | | | WO | 02096261 A2 | 05-12-2002 |

EPO FORM P0459

For more details about this annex : see Official Journal of the European Patent Office, No. 12/82